# EUROPÄISCHE PATENTSCHRIFT

(11) **EP 0 993 458 B1**
(45) Veröffentlichungstag und Bekanntmachung des Hinweises auf die Patenterteilung: **09.04.2003**
(21) Anmeldenummer: 98932135.1
(22) Anmeldetag: 08.06.1998
(51) Int. Cl.: C07D 401/06, A61K 31/445, C07D 491/04

(54) **3-BENZYLPIPERIDINE**
3-BENZYLPIPERIDINE
3-BENZYLPIPERIDINES

(30) Priorität: 18.06.1997 DE 19725664
(43) Veröffentlichungstag der Anmeldung: 19.04.2000
(73) Patentinhaber: MERCK PATENT GmbH, 64293 Darmstadt (DE)
(72) Erfinder: BÖTTCHER, Henning, D-64287 Darmstadt (DE); PRÜCHER, Helmut, D-64646 Heppenheim (DE); GREINER, Hartmut, D-64331 Weiterstadt (DE); SEYFRIED, Christoph, D-64342 Seeheim-Jugenheim (DE); BARBER, Andrew, D-64331 Weiterstadt (DE); MARTINEZ, Joseph-Maria, E-08080 Barcelona (ES)
(86) Internationale Anmeldenummer: EP9803429
(87) Internationale Veröffentlichungsnummer: WO98057953

(56) Entgegenhaltungen:
- EP-A- 0 518 805
- WO-A-94/24105
- WO-A-95/02592
- WO-A-95/33743
- US-A- 4 251 538
- US-A- 5 116 846
- US-A- 5 256 673

## Beschreibung

Die Erfindung betrifft Verbindungen der Formel I 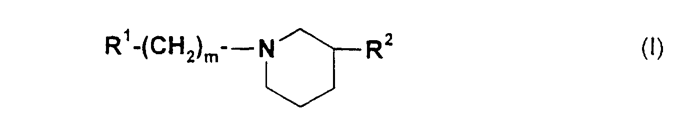 worin
- R¹: unsubstituiertes oder ein- oder zweifach durch Hal, CN, A, AO, OH, CONH₂, CONHA, CONA₂, COOH und/oder COOA substituiertes 2-oder 3-Indolyl, 5H-1,3-Dioxolo-[4,5-f]-7-indolyl,
- R²: unsubstituiertes oder ein-, zwei- oder dreifach durch A, AO, OH, Hal, CN, NO₂, NH₂, NHA, NA₂, CF₃ COA, CONH₂, COOH, CONHA, CONA₂, OSO₂A und/oder OSO₂CF₃ substituiertes Benzyl oder Phenylhydroxy-methyl,
- Hal: F, Cl, Br, oder I,
- A: geradkettiges oder verzweigtes Alkyl mit 1-10 C-Atomen, das durch 1 bis 5 F- und/oder Cl-Atome substituiert sein kann oder Cycloalkyl mit 3-10 C-Atomen;
und
- m: 4
bedeuten, sowie deren physiologisch unbedenklichen Salze und Solvate.

Verbindungen mit ähnlicher Struktur sind beispielsweise aus den Dokumenten WO 95/02592 oder WO 95/33743 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde nun gefunden, dass die Verbindungen der Formel I und ihre Salze bei guter Vertäglichkeit besonders wertvolle pharmakologische Eigenschaften besitzen. Sie zeigen Wirkungen auf das Zentralnervensystem und haben potente anti-epileptische und vor allem antiischämische Eigenschaften (dies wurde im Modell der transienten Okklusion der mittleren Zerebralarterie bei Ratten und der Reduktion der neurologischen Scores gezeigt). Diese Substanzen stellen potente σ-Rezeptor Liganden mit neuroprotektiven Eigenschaften dar (M. O'Neill et al., European J. Pharmacol. 283(1995), 217-225). Verbindungen, die als σ-Rezeptor Liganden wirken, beeinflussen auch den NMDA-lonen-Kanal-Komplex (siehe auch H. Yamamoto et al., J. Neuroscience (1995), 15(1), 731-736). σ-Agonisten haben zusätzlich einen günstigen Einfluss auf altersbedingte Erinnerungsstörungen (vgl. Maurice et al., Brain Research 733 (1996), 219-230). Einige der Verbindungen der Formel I zeigen ferner eine starke 5-HT-reuptake-hemmende Wirkung. Für solche Verbindungen ist eine besonders gute antidepressive, anxiolytische Wirkung, sowie eine positive Beeinflussung von Zwangsverhalten (OCD), Eßstörungen wie Bulimie, tardiver Dyskinesien, Lernstörungen, altersabhängiger Erinnerungsstörungen als auch von Psychosen zu erwarten.

Verbindungen der Formel I und ihre physiologisch unbedenklichen Säureadditionssalze weisen bei guter Verträglichkeit besonders wertvolle pharmakologische Eigenschaften auf. Die Verbindungen sind geeignet für die Behandlung von Schizophrenie, kognitiven Defiziten, Angst, Depressionen, Übelkeit, tardive Dyskinesie, Magen-Darm-Trakt-Störungen oder Parkinsonismus. Sie zeigen Wirkungen auf das Zentralnervensystem, vor allem 5-HT-reuptake hemmende Wirkungen. Zum ex-vivo Nachweis der Serotoninwiederaufnahmehemmung wird die synaptosomale Aufnahmehemmung (Wong et al., Neuropsychopharmacol. 8 (1993), 23-33 und der p-Chloramphetaminantagonismus (Füller et al., J. Pharmacol. Exp. Ther. 212 (1980), 115-119) herangezogen.

Da diese Substanzen als σ-Liganden eine neuroprotektive Wirkung besitzen, sind sie auch insbesondere als Therapeutika zur Schlaganfallbehandlung, zur Behandlung von Hirn- und Rückenmarkstraumata, wie auch zur Behandlung von ischämischen Zuständen nach einem Herzstillstand geeignet.

Die Verbindungen der Formel I eignen sich daher sowohl in der Veterinärais auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Sind sie jedoch auch geeignet als Arzneimittelwirkstoffe für Anxiolytika, Antidepressiva, Antipsychotika, Neuroleptika, Antihypertonika und/oder zur positiven Beeinflussung von Zwangsverhalten, Schlafstörungen, tardiver Dyskinesien, Lernstörungen, alterabhängiger Erinnerungsstörungen, Eßstörungen wie Bulimie und/oder Sexualfunktionsstörungen.

Gegenstand der Erfindung sind somit die Verbindungen der Formel I sowie ihre physiologisch unbedenklichen Säureadditionssalze.

Gegenstand der Erfindung sind insbesondere Verbindungen der Formel I, ausgewählt aus der Gruppe
a) 3-[4-(3-Benzyl-piperidin-1-yl]-butyl}-indol;
b) 3-[4-(3-Phenyl-hydroxyphenyl-piperidin-1-yl)-butyl]-indol;
c) 3-[4-(3-Benzyl-piperidin-1-yl)-butyl]-5-fluor-indol;
d) 3-[4-(3-Benzyl-piperidin-1-yl)-butyl]-5-carboxy-indol;
e) 3-[4-(3-Benzyl-piperidin-1-yl)-butyl]-indol-5-carbonsäuremethylester;
f) (-)-3-{4-[3-(3R')-Benzyl-piperidin-1-yl]-butyl}-indol-5-carbonsäuremethylester;
g) (+)-3-{4-[3-(3S')-Benzyl-piperidin-1-yl]-butyl}-indol-5-carbonsäuremethylester;
h) 3-{4-[3-(3R')-Benzyl-piperidin-1-yl]-butyl}-6-methoxyindol;
i) 3-{4-[3-(3S')-Benzyl-piperidin-1-yl]-butyl}-6-methoxyindol;
j) (+)-3-[4-(3-Benzyl-piperidin-1-yl)-butyl]-indol;
k) (-)-3-[4-(3-Benzyl-piperidin-1-yl)-butyl]-indol;
l) 3-[4-(3-Benzyl-piperidin-1-yl)-butyl]-5-chlorindol;
m) 3-[4-(3-Benzyl-piperidin-1-yl)-butyl]-5-methoxy-indol;
n) 3-{4-[3-(4-Fluorbenzyl}-piperidin-1-yl]-butyl}-5-fluor-indol;
o) 7-{4-[(3R)-3-Benzyl-piperidin-1-yl]-butyl}-5H-1,3-dioxolo[4,5-f]indol;
p) 7-{4-[(3S)-3-Benzyl-piperidin-1-yl]-butyl}-5H-1,3-dioxolo[4,5-f]indol;
q) 3-{4-[(3R)-3-Benzyl-piperidin-1-yl]-butyl}-5-fluorindol;
r) 3-{4-[(3S)-3-Benzyl-piperidin-1-yl]-butyl}-5-fluorindol;
s) 3-{4-[(3R)-3-Benzyl-piperidin-1-yl]-butyl}-indol-5-carbonitril;
t) 3-{4-[(3S)-3-Benzyl-piperidin-1-yl]-butyl}-indol-5-carbonitril;
u) 3-{4-[3-(4-Fluorphenylhydroxymethyl)-piperidin-1-yl]-butyl}-indol-5-carbonitril;
sowie deren Salze und Solvate.

Für alle Reste, die mehrfach auftreten, wie z.B. A, gilt, daß deren Bedeutungen unabhängig voneinander sind.

Solvate sind z.B. Hemi-, Mono- oder Dihydrate oder beispielsweise Additionsverbindungen mit Alkoholen.

Der Rest A bedeutet Alkyl und hat 1 bis 10 , vorzugsweise 1, 2, 3, 4, 5 oder 6, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, ferner auch Fluormethyl, Difluormethyl, Trifluormethyl, 1,1,1-Trichlorethyl oder Pentafluorethyl.

Cycloalkyl bedeutet insbesondere z.B. Cyclopropyl, Cyclobutyl, Cyclopentyl, Cyclohexyl, Cycloheptyl oder 1-Adamantyl.

OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, Isopropylamino, n-Butylamino, Isobutylamino, sek.-Butylamino oder tert.-Butylamino. NA₂ bedeutet vorzugsweise Dimethylamino, ferner N-Ethyl-N-methyl-amino, Diethylamino, Di-n-propylamino, Diisopropylamino oder Di-n-butylamino. Daraus resultierend bedeutet CO-NHA vorzugsweise N-Methylcarbamoyl oder N-Ethylcarbamoyl; CO-NA₂ vorzugsweise N,N-Dimethylcarbamoyl oder N,N-Diethylcarbamoyl.

Hal bedeutet Fluor, Chlor, Brom oder lod, insbesondere Fluor oder Chlor.

Der Rest R¹ bedeutet vorzugsweise unsubstituiertes oder ein- oder zweifach, insbesondere jedoch einfach, durch Hal, CN, A, AO, OH, CONH₂, CONHA, CONA₂, COOH und/oder COOA substituiertes 2- oder 3-Indolyl, ferner auch 5H-1,3-Dioxolo[4,5-f]-7-indolyl, welches ebenfalls unsubstituiert, ein- oder zweifach vorzugsweise durch Hal, CN, A, AO oder OH substituiert sein kann, insbesondere liegt dieser Rest jedoch unsubstituiert vor.

R¹ bedeutet daher vorzugsweise 2- oder 3-Indolyl, 5- oder 6-Methyl-indol-2-yl, 5- oder 6-Methyl-indol-3-yl, 5- oder 6-Methoxy-indol-2-yl, 5- oder 6-Methoxy-indol-3-yl, 5- oder 6-Hydroxy-indol-2-yl, 5- oder 6-Hydroxyindol-3-yl, 5- oder 6-Fluor-indol-2-yl, 5- oder 6-Fluor-indol-3-yl, 5- oder 6-Cyan-indol-2-yl, 5- oder 6-Cyan-indol-3-yl, 5- oder 6-Chlor-indol-2-yl, 5-oder 6-Chlor-indol-3-yl, 5- oder 6-Carboxy-indol-2-yl, 5- oder 6-Carboxyindol-3-yl, 5- oder 6-Methoxycarbonyl-indol-2-yl, 5- oder 6-Methoxycarbonyl-indol-3-yl, 5H-1,3-Dioxolo[4,5-f]-indol-7-yl, ferner 5- oder 6-Bromindol-2-yl, 5- oder 6-Brom-indol-3-yl, 5- oder 6-Ethyl-indol-2-yl, 5- oder 6-Ethyl-indol-3-yl, 5- oder 6-Trifluormethyl-indol-2-yl, 5- oder 6-Trifluormethyl-indol-3-yl, 5- oder 6-Isopropyl-indol-2-yl, 5- oder 6-Isopropylindol-3-yl, 5- oder 6-Dimethylamino-indol-3-yl oder 5- oder 6-Dimethylamino-indol-2-yl, 5- oder 6-Ethoxy-indol-3-yl oder 5- oder 6-Ethoxy-indol-2-yl.

Der Rest R² bedeutet vorzugsweise unsubstituiertes oder ein-, zwei- oder dreifach durch Hal, OH, OA, A, NH₂, NHA, NA₂, CONH₂, CONHA, CONA₂, COOH, COA, CF₃, CN, OSO₂A, OSO₂CF₃ und/oder NO₂ substituiertes Benzyl oder Phenylhydroxymethyl. R² bedeutet demnach insbesondere bevorzugt Benzyl, Phenylhydroxymethyl und einfach durch Hal substituiertes Benzyl oder Phenylhydroxymethyl, also z.B. p-Fluorbenzyl oder p-Fluorphenylhydroxylmethyl, p-Methyl-benzyl oder p-Methylphenylhydroxymethyl, p-Chlorbenzyl oder p-Chlorphenylhydroxymethyl. Femer bevorzugt bedeutet R² p-Aminobenzyl, p-Methylaminobenzyl, p-Dimethylaminobenzyl, p-Ethylaminobenzyl, p-Cyanobenzyl, m-Fluorbenzyl, m-Methylbenzyl oder m-Methylphenylhydroxymethyl, p-Nitrobenzyl oder p-Nitrophenylhydroxymethyl.

Für die gesamte Erfindung gilt, daß sämtliche Reste, die in einem Molekül mehrfach auftreten können, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln la bis Ik ausgedrückt werden, die der Formel I entsprechen und worin die nicht näher bezeichneten Reste die bei der Formel I angegebene Bedeutung haben, worin jedoch
- in la: R¹ unsubstituiertes 3-Indolyl ist;
- in Ib: R¹ in 5-Position substituiertes 3-Indoiyl ist;
- in le: R¹ eine in Ib angegebene Bedeutung hat und der Substituent Hal,
Methoxycarbonyl, CN oder Carboxy ist;
- in If: R¹ 5H-1,3-Dioxolo[4,5-f]-indol-7-yl ist;
- in Ig: R² unsubstituiertes Benzyl bedeutet;
- in Ih: R² eine in Ig angegebene Bedeutung hat, wobei der Benzylring jedoch einfach substituiert vorliegt;
- in li: R² eine in Ih angegebene Bedeutung hat und der Substituent Hal ist;
- in Ij: R² unsubtituiertes oder einfach substituiertes Phenylhydroxymethyl ist;
- in Ik: R² eine in Ij angegebene Bedeutung hat und der Substituent Hal ist.

Gegenstand der Erfindung ist ferner ein Verfahren zur Herstellung von Verbindungen der Formel sowie von deren Salzen und Solvaten, dadurch gekennzeichnet, dass man
eine Verbindung der Formel II 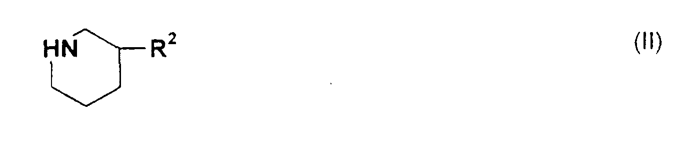 worin R² die angegebene Bedeutung hat, mit einer Verbindung der Formel III

R¹-(CH₂)ₘ-L (III)

worin
- L: Cl, Br, I, OH, OCOA, OCOPh, OSO₂A, OSO₂Ar,
wobei Ar für Phenyl oder Tolyl und A für Alkyl stehen, oder eine andere reaktionsfähig veresterte OH-Gruppe oder leicht nucleophil substituierbare Abgangsgruppe bedeutet
und
- R¹ und m: die in Anspruch 1 angegebenen Bedeutungen haben,
umsetzt,
oder
in einer reduktiven Aminierung eine Verbindung der Formel IV

R¹ - (CH₂)ₘ₋₁ - CHO (IV)

worin R¹ und m die in Anspruch 1 angegebenen Bedeutungen haben, mit einer Verbindung der Formel II umsetzt,
oder dass man eine sonst der Formel I entsprechende Verbindung, die jedoch anstelle eines oder mehrerer Wasserstoffatome eine oder mehrere reduzierbare Gruppe(n) wie C=O und/oder eine oder mehrere zusätzliche C-C- und/oder C-N-Bindung(en) enthält, mit einem reduzierenden Mittel behandelt,
oder dass man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder dass man gegebenenfalls einen Rest R¹ und/oder R² in einen anderen Rest R¹ und/oder R² umwandelt, indem man z.B. eine OA-Gruppe unter Bildung einer OH-Gruppe spaltet und/oder eine CN-, COOH-, COOA-Gruppe derivatisiert und/oder dass man z.B. ein primäres oder sekundäres N-Atom alkyliert und/oder eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 41 01 686) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden vorzugsweise erhalten, indem man Verbindungen der Formel II mit Verbindungen der Formel III umsetzt. Die Ausgangsstoffe der Formeln II und III sind in der Regel bekannt; die nicht bekannten Verbindungen der Formeln II und III können leicht analog zu den bekannten Verbindungen hergestellt werden.

Die Piperidin-Derivate der Formel II sind größtenteils bekannt. Sofern sie nicht käuflich zu erwerben oder bekannt sind, können sie nach an sich bekannten Methoden hergestellt werden.

Die Indol-Derivate der Formel III sind größtenteils bekannt und teilweise auch käuflich zu erwerben.Ferner kann man die Verbindungen durch elektrophile oder in bestimmten Fällen auch nukleophile aromatische Substitutionen aus bekannten Verbindungen herstellen. Als Ausgangssubstanz wird vorzugsweise eine entsprechende Indol-3-alkansäure (herstellbar analog einer Japp-Klingemann-Typ Fischer Indolsynthese, vgl. dazu Böttcher et al., J. Med. Chem. 1992, 35, 4020-4026 oder lyer et al., J. Chem. Soc. Perkin Trans. II 1973, 872-878) verwendet. Die Säuregruppe wird dann durch Einführung der reaktionsfähigen Abgangsgruppe L, eventuell nach vorheriger Reduktion der Säuregruppe nach bekannten Methoden z.B. mit Lithiumaluminiumhydrid in Tetrahydrofuran zur OH-Gruppe, für die Umsetzung mit dem Piperidin-Derivat vorbereitet.

Die Umsetzung der Verbindungen II und III verläuft nach Methoden, wie sie für die Alkylierung bzw. Acylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidion (NMP); Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Gegebenenfalls ist es notwendig, vor der Durchführung dieser Reaktion weitere enthaltene Aminogruppen durch Einführung von geeigneten Schutzgruppen vor einer Alkylierung oder Acylierung zu schützen. Der Ausdruck Aminoschutzgruppe ist allgemein bekannt und bezieht sich auf Gruppen, die geeignet sind, eine Aminogruppe vor chemischen Umsetzungen zu schützen, die aber leicht entfernbar sind, nachdem die gewünschte chemische Reaktion an einer anderen Stelle des Moleküls durchgeführt worden ist. Da dem Fachmann solche Schutzgruppen sowie die Einführung und Abspaltung dieser aus zahlreichen Literaturstellen und Lehrbüchern wohl bekannt sind, muss an dieser Stelle darauf nicht näher eingegangen werden.

Verbindungen der Formel I lassen sich weiterhin durch reduktive Aminierung von Verbindungen der Formel IV mit Verbindungen der Formel II erhalten. Die Ausgangsstoffe der Formeln IV und II sind teilweise bekannt. Sofern sie nicht bekannt sind, können sie nach an sich bekannten Methoden hergestellt werden.

Die reduzierende Aminierung kann in Gegenwart von Reduktionsmitteln wie zum Beispiel NaBH₃CN und NaBH(OAc)₃ durchgeführt werden.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe I, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall gelöst in einem Alkohol wie Ethanol, Isopropanol, Butanol, Amyl- oder Isoamylalkohol oder Phenol. Man kann ferner eine Aluminium-Nickel-Legierung in alkalisch-wäßriger Lösung, gegebenenfalls unter Zusatz von Ethanol, verwenden. Auch Natrium- oder Aluminiumamalgam in wäßrig-alkoholischer oder wäßriger Lösung ist zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner besonders vorteilhaft komplexe Metallhydride, wie LiAlH₄, NaBH₄, Diisobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für die Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden mit LiAlH₄ in THF bei Temperaturen zwischen etwa 0 und 66° zu CH₂-Gruppen reduzieren.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 250° Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel wie Diethylenglykol oder Triethylenglykol, in Gegenwart von Alkali, wie Natriumhydroxid, umsetzt. Das Reaktionsgemisch wird in der Regel etwa 3-4 Stunden gekocht.

Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Weiterhin kann man eine Verbindung der Formel I nach an sich bekannten Methoden in eine andere Verbindung der Formel I umwandeln.

Verbindungen der Formel I, worin R¹ einen durch CONH₂, CONHA oder CONA₂ substituierten Rest bedeutet, können durch Derivatisierung entsprechender substituierter Verbindungen der Formel I durch partielle Hydrolyse erhalten werden. Ferner ist es möglich, Cyan-substituierte Verbindungen der Formel I zunächst zu Säuren zu hydrolysieren und die Säuren mit primären oder sekundären Aminen zu amidieren. Bevorzugte ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem haloenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°

Besonders günstig ist es aber auch in umgekehrter Weise, durch Wasserabspaltung, ausgehend von den Amiden, z.B. mittels Trichloracetylchlorid/Et₃N [Synthesis (2), 184 (1985)] oder mit POCl₃ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen.

In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Erfindungsgemäße Verbindungen der Formel I können aufgrund ihrer Molekülstruktur chiral sein und können dementsprechend in zwei enantiomeren oder mehreren diastereomeren Formen auftreten. Sie können daher aufgrund von einem oder mehrerer chiraler Zentren in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenwert sein, die Diastereomere bzw. Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kiesegel fixierte chiral derivatisierte Methacrylatpolymere). Als Laufmittel eignen sich hierfür wäßrige oder alkoholische Lösungsmittelgemische.

Im Falle racemischer Säuren können in analoger Weise optische aktive Basen, wie z.B. die R- und S-Form von 1-Phenylethylamin, 1-Naphthylethylamin, Dihydroabietylamin, Cinchonin oder Cinchonidine verwendet werden.

Unter besonderen Bedingungen ist es aber auch bereits während der Synthese möglich, entsprechende enantiomerenreine Zwischenprodukte einzusetzen, welche nach einem der oben erwähnten Verfahen hergestellt wurden. Dabei bleibt die Chiralität im Verlauf der weiteren Synthese erhalten.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden.

Sie eignen sich zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesie, Parkinsonismus und/oder Psychosen und von Nebenwirkungen bei der Behandlung der Hypertonie (z.B. mit α-Methyldopa). Femer können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide.

Insbesondere bevorzugt können sie auch als Therapeutika zur Bekämpfung der Folgen cerebraler Infarktgeschehen (Apoplexia cerebri), wie Schlaganfall und cerebraler Ischämien und zur Behandlung von Hirnund Rückenmarkstraumata eingesetzt werden.

Dabei werden die erfindungsgemäßen Substanzen in der Regel in Analogie zu ähnlich wirkenden bekannten Präparaten verabreicht, vorzugsweise in Dosierungen zwischen etwa 0,1 und 500 mg, insbesondere zwischen 0,2 und 300 mg pro Dosierungseinheit. Die tägliche Dosierung liegt vorzugsweise zwischen etwa 0,001 und 250 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, dampft ein und reinigt durch Chromatographie an Kieselgel, wobei auch eine Trennung der nachfolgend beschriebenen Isomeren erfolgt, und/oder durch Kristallisation. R_{f}-Werte wurden dünnschichtchromatographisch an Kieselgel erhalten. M⁺+1-Werte wurden durch FAB-MS (**F**ast-**A**tom-**B**ombardment-**M**assen-**S**pektroskopie) ermittelt. Die angegebenen Drehwerte der optisch aktiven Verbindungen wurden jeweils an Lösungen der freien Basen gemessen.

### Beispiel 1

a) Eine Lösung von 4-(5-Fluor-indol-3-yl)-butansäure [erhältlich z.B. durch Diazotierung des entsprechend substiuierten Anilins, Kondensierung des Diazoniumsalzes mit 1-Oxocyclohexan-2-carbonsäurethylester nach der Japp-Klingemann-Methode zu der entsprechenden 4-(5-Fluor-2-ethoxycarbonyl-indol-3-yl)-butansäure und anschließender Verseifung und Decarboxylierung der Ethoxycarbonylgruppe] in THF wird mit Lithiumaluminiumhydrid einer Reduktion unterworfen. Nach üblicher Aufarbeitung erhält man 4-(5-Fluor-indol-3-yl)-butanol.
   Dieses wird durch Umsetzung mit Methansulfonsäurechlorid in den reaktionsfähigen Methansulfonsäure-4-(5-fluor-indol-3-yl)butylester überführt.
b) Ein Gemisch aus 2.80 g (0.0098 Mol) Methansulfonsäure-4-(5-fluorindol-3-yl)-butylester, 3.20 g (0.0097 Mol) (S)-(+)-(Mandelsäure/3-Benzylpiperidin), 3.80 g (0.0294 Mol) Ethyl-diisopropylamin wird in 100 ml Acetonitril ca. 96 Stunden auf dem Dampfbad erhitzt. Das Reaktionsgemisch wird dann filtriert und eingeengt, der Rückstand in Ethylacetat aufgelöst, mit Wasser extrahiert. Die organische Phase wird aufgearbeitet, und der Rückstand in Ethylacetat über eine Kieselgelsäule chromatographiert. Die Fraktionen wurden eingeengt, der Rückstand in Ethylacetat gelöst und mit ethanolischer HCI das Hydrochlorid des 3-{4-[(3S)-3-Benzyl-piperidin-1-yl]-butyl}-5-fluorindols gefällt und aufgearbeitet, F. 152.0-153.0 °C, α_{D}²⁰ (+) 9.7° (c 1.0, Dimethylsulfoxid).

Analog erhält man durch Umsetzung von
Methansulfonsäure-4-(5-fluor-indol-3-yl)butylester mit
(R)-(-)-(3-Benzylpiperidin)-mandelat
das entsprechende Hydrochlorid des
3-{4-[(3R)-3-Benzyl-piperidin-1-yl]-butyl}-5-fluorindol mit F. 153.0-154.0°, α_{D}²⁰ (-) 10.8° (c 1.0, Dimethylsulfoxid).

In analoger Weise erhält man:
(+)-3-[4-(3-Benzyl-piperidin-1-yl)-butyl]-indol, F. 74-75°, α_{D}²⁰ (+) 22.1° (c 1.0, Dimethylsulfoxid);
(-)-3-[4-(3-Benzyl-piperidin-1-yl)-butyl]-indol, F. 73-74°, α_{D}²⁰(-) 22.8° (c 1.0, Dimethylsulfoxid);
(+)-3-{4-[3-(3S')-Benzyl-piperidin-1-yl]-butyl}-indol-5-carbonsäuremethylester; α_{D}²⁰ (+) 60° (c 1.0, Dimethylsulfoxid);
(-)-3-{4-[3-(3R')-Benzyl-piperidin-1-yl]-butyl}-indol-5-carbonsäuremethylester; α_{D}²⁰ (-) 8.1° (c 1.0, Dimethylsulfoxid);
3-{4-[(3R')-3-Benzyl-piperidin-1-yl]-butyl}-6-methoxy-indol, Hydrochlorid, F. 218-221°, α_{D}²⁰ (-) 7.3° (c 1.0, Dimethylsulfoxid);
3-{4-[(3S')-3-Benzyl-piperidin-1-yl]-butyl}-6-methoxy-indol, Hydrochlorid, F. 217-220°, α_{D}²⁰ (+) 75° (c 1.0, Dimethylsulfoxid).

### Beispiel 2

Analog Beispiel 1 a) und b), jedoch ohne im Schritt b) die Mandelsäure zuzusetzen, erhält man ausgehend von 4-(5-Fluor-indol-3-yl)butansäure über Methansulfonsäure-4-(5-fluor-indol-3-yl)butylester durch Umsetzung mit 3-Benzylpiperidin das 3-[4-(3-Benzyl-piperidin-1-yl)-butyl]-5-fluor-indol, Hydrochlorid, F. 163-165°

Analog erhält man durch Umsetzung von
Methansulfonsäure-4-(indol-3-yl)butylester mit 3-Benzylpiperidin das 3-[4-(3-Benzyl-piperidin-1-yl)-butyl]-indol, Hydrochlorid, F. 134-136°;
Methansulfonsäure-4-(5-chlor-indol-3-yl)butylester mit 3-Benzyliperidin das 3-[4-(3-Benzyl-piperidin-1-yl)-butyl]-5-chlor-indol, Malonat, F. 127-129°;
Methansulfonsäure-4-(5-fluor-indol-3-yl)butylester mit 3-(4-Fluorenzyl)piperidin das 3-{4-[3(4-Fluorbenzyl)-piperidin-1-yl]-butyl}-5-fluor-indol.

### Beispiel 3

Eine Lösung aus äquivalente Mengen 3-Benzylpiperidin und 3-(4-Chlorbutyl)-5-cyan-indol (erhältlich z.B. durch Halogenierung der entsprechenden Alkoholverbindung, welche analog zu Beispiel 1a) durch Reduktion aus der 4-(5-Cyan-indol-3-yl)butansäure hergestellt werden kann) in Acetonitril wird bei Raumtemperatur ca. 6 Stunden gerührt. Nach üblicher Aufarbeitung und Auftrennung der chiralen Verbindungen erhält man das 3-{4-[(3S)-3-Benzyl-piperidin-3-yl]-butyl}-5-cyan-indol, α_{D}²⁰ (+) 8.9° (c 1.0, Dimethylsulfoxid), als Hydrochlorid, F. 65-67° (amorph); bzw. das entsprechende 3-{4-[(3R)-3-Benzyl-piperidin-3-yl]-butyl}-5-cyan-indol, α_{D}²⁰ (-) 10.4° (c 1.0, Dimethylsulfoxid), als Hydrochlorid, F. 65-75° (amorph).

Analog können hergestellt werden:
7-{4-[(3R)-3-Benzyl-piperidin-1-yl]-butyl}-5H-1,3-dioxolo[4,5-f]indol, R_{f} 0.4 (in CHCl₃:MeOH 95:5);
7-{4-[(3S)-3-Benzyl-piperidin-1-yl]-butyl}-5H-1,3-dioxolo[4,5-f]indol, R_{f} 0.4 (in CHCl₃:MeOH 95:5).

### Beispiel 4

Äquimolare Mengen 3-Benzylpiperidin und 4-(5-Methoxy-indol-3-yl)butansäure (Herstellung analog Beispiel 1a) werden unter Zugabe von 2-Chlor-1-methylpyridiniumiodid werden in THF bei Raumtemperatur umgesetzt. Nach üblicher Aufarbeitung erhält man 3-Benzyl-1-[4-(5-methoxy-indol-3-yl)-butanoyl]-piperidin.

Analog werden hergestellt:
3-Benzyl-1-[4-(5-fluor-indol-3-yl)-butanoyl]-piperidin;
3-Benzyl-1-[4-(5-chlor-indol-3-yl)-butanoyl]-piperidin;
3-Benzyl-1-[4-(6-chlor-indol-3-yl)-butanoyl]-piperidin;
3-Benzyl-1-[4-(6-fluor-indol-3-yl)-butanoyl]-piperidin;
3-Benzyl-1-[4-(6-methoxy-indol-3-yl)-butanoyl]-piperidin;
3-Benzyl-1-[4-(6-ethoxy-indol-3-yl)-butanoyl]-piperidin;
3-Benzyl-1-[4-(5-ethoxy-indol-3-yl)-butanoyl]-piperidin;
3-Benzyl-1-[4-(5-cyan-indol-3-yl)-butanoyl]-piperidin;
3-Benzyl-1-[4-(6-cyan-indol-3-yl)-butanoyl]-piperidin;
3-Benzyl-1-[4-(5-fluor-indol-2-yl)-butanoyl]-piperidin;
3-Benzyl-1-[4-(5-chlor-indol-2-yl)-butanoyl]-piperidin;
3-Benzyl-1-[4-(6-chlor-indol-2-yl)-butanoyl]-piperidin;
3-Benzyl-1-[4-(6-fluor-indol-2-yl)-butanoyl]-piperidin;
3-Benzyl-1-[4-(6-methoxy-indol-2-yl)-butanoyl]-piperidin;
3-Benzyl-1-[4-(6-ethoxy-indol-2-yl)-butanoyl]-piperidin;
3-Benzyl-1-[4-(5-ethoxy-indol-2-yl)-butanoyl]-piperidin;
3-Benzyl-1-[4-(5-cyan-indol-2-yl)-butanoyl]-piperidin;
3-Benzyl-1-[4-(6-cyan-indol-2-yl)-butanoyl]-piperidin.

### Beispiel 5

3-Benzyl-1-[4-(5-methoxy-indol-3-yl)-butanoyl]-piperidin wird in THF mit Lithiumaluminiumhydrid umgesetzt. Nach üblicher Aufbarbeitung erhält man das 3-[4-(3-Benzyl-piperidin-1-yl)-butyl]-5-methoxy-indol, Hydrochlorid F. 222-224°.

Analog können hergestellt werden:
3-[4-(3-Benzyl-piperidin-1 -yl)-butyl]-5-fluor-indol;
3-[4-(3-Benzyl-piperidin-1-yl)-butyl]-5-chlor-indol;
3-[4-(3-Benzyl-piperidin-1-yl)-butyl]-6-chlor-indol;
3-[4-(3-Benzyl-piperidin-1-yl)-butyl]-6-fluor-indol;
3-[4-(3-Benzyl-piperidin-1-yl)-butyl]-6-methoxy-indol;
3-[4-(3-Benzyl-piperidin-1-yl)-butyl]-6-ethoxy-indol;
3-[4-(3-Benzyl-piperidin-1-yl)-butyl]-5-ethoxy-indol;
3-[4-(3-Benzyl-piperidin-1-yl)-butyl]-5-cyan-indol;
3-[4-(3-Benzyl-piperidin-1-yl)-butyl]-6-cyan-indol;
2-[4-(3-Benzyl-piperidin-1-yl)-butyl]-5-fluor-indol;
2-[4-(3-Benzyl-piperidin-1-yl)-butyl]-5-chlor-indol;
2-[4-(3-Benzyl-piperidin-1-yl)-butyl]-6-chlor-indol;
2-[4-(3-Benzyl-piperidin-1-yl)-butyl]-6-fluor-indol;
2-[4-(3-Benzyl-piperidin-1-yl)-butyl]-6-methoxy-indol;
2-[4-(3-Benzyl-piperidin-1-yl)-butyl]-6-ethoxy-indol;
2-[4-(3-Benzyl-piperidin-1-yl)-butyl]-5-ethoxy-indol;
2-[4-(3-Benzyl-piperidin-1-yl)-butyl]-5-cyan-indol;
2-[4-(3-Benzyl-piperidin-1-yl)-butyl]-6-cyan-indol.

### Beispiel 6

3-[4-(3-Benzoyl-piperidin-1-yl)-butyl]-indol wird durch Umsetzung in THF mit Natriumborhydrid einer Reduktion unterworfen. Nach üblicher Aufarbeitung erhält man 3-[4-(3-Phenyl-hydroxymethyl-piperidin-1-yl)-butyl]-indol, Hydrochlorid, F. 181-183°.

Analog werden hergestellt:
3-[4-(3-Phenyl-hydroxymethyl-piperidin-1-yl)-butyl]-5-fluor-indol;
3-{4-[3-(3R')-Phenyl-hydroxymethyl-piperidin-1-yl]-butyl}-6-methoxy-indol;
3-{4-[3-(3S')-Phenyl-hydroxymethyl-piperidin-1-yl]-butyl}-6-methoxy-indol;
3-[4-(3-Phenyl-hydroxymethyl-piperidin-1-yl)-butyl]-5-chlor-indol;
3-[4-(3-Phenyl-hydroxymethyl-piperidin-1-yl)-butyl]-5-methoxy-indol;
3-{4-[3-(4-Fluor-phenyl-hydroxymethyl)-piperidin-1-yl]-butyl}-5-fluor-indol;
3-{4-[(3R)-3-Phenyl-hydroxymethyl-piperidin-1-yl]-butyl}-5-fluorindol;
3-{4-[(3S)-3-Phenyl-hydroxymethyl-piperidin-1-yl]-butyl}-5-fluorindol;
3-{4-[(3R)-3-Phenyl-hydroxymethyl-piperidin-1-yl]-butyl}-indol-5-carbonitril;
3-{4-[(3S)-3-Phenyl-hydroxymethyl-piperidin-1-yl]-butyl}-indol-5-carbonitril;
3-{4-[3-(4-Fluor-phenylhydroxymethyl)-piperidin-1-yl]-butyl}-indol-5-carbonitril, F. 157-159°.

### Beispiel 7

Ein Gemisch aus 0.0098 Mol Methansulfonsäure-4-(5-carbonsäuremethylester-indol-3-yl)-butylester (Herstellung analog Beispiel 1a) und 0.0097 Mol 3-Benzylpiperidin wird analog Beispiel 1b in Acetonitril ca. 96 Stunden auf dem Dampfbad erhitzt. Das Reaktionsgemisch wird wie beschrieben aufgearbeitet und gereinigt. Man erhält so den 3-[4-(3-Benzyl-piperidin-1-yl)-butyl]-indol-5-carbonsäuremethylester, Hydrochlorid, F. 181-183°.

Analog können hergestellt werden:
3-{4-[3-(4-Fluorbenzyl)-piperidin-1-yl]-butyl}-indol-5-carbonsäuremethylester;
3-{4-[3-(4-Cyanbenzyl)-piperidin-1-yl]-butyl}-indol-5-carbonsäuremethylester;
3-{4-[3-(4-Ethylbenzyl)-piperidin-1-yl]-butyl}-indol-5-carbonsäuremethylester;
3-{4-[3-(4-Methylbenzyl)-piperidin-1-yl]-butyl}-indol-5-carbonsäuremethylester;
3-{4-[3-(4-Methoxybenzyl)-piperidin-1-yl]-butyl}-indol-5-carbonsäuremethylester;
3-{4-[3-(4-Fluorbenzyl)-piperidin-1-yl]-butyl}-indol-6-carbonsäuremethylester;
3-{4-[3-(4-Fluorbenzyl)-piperidin-1-yl]-butyl}-indol-5-carbonsäuremethylester
3-[4-(3-Benzyl-piperidin-1-yl)-butyl]-indol-6-carbonsäuremethylester
3-{4-[3-(4-Cyanbenzyl)-piperidin-1-yl]-butyl}-indol-6-carbonsäuremethylester
3-{4-[3-(4-Methylbenzyl)-piperidin-1-yl]-butyl}-indol-6-carbonsäuremethylester.

### Beispiel 8

Ein Gemisch aus 3-[4-(3-Benzyl-piperidin-1-yl)-butyl]-indol-5-carbonsäuremethylester und KOH wird in Ethanol unter Rückfluss erhitzt. Nach üblicher Aufarbeitung erhält man 3-[4-(3-Benzyl-piperidin-1-yl)-butyl]-5-carboxy-indol, Hydrat, F. 144-147°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I 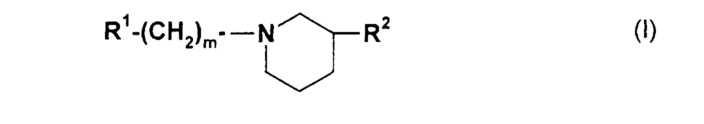 worin
R¹ unsubstituiertes oder ein- oder zweifach durch Hal, CN, A, AO, OH, CONH₂, CONHA, CONA₂, COOH und/oder COOA substituiertes 2-oder 3-Indolyl, 5H-1,3-Dioxolo[4,5-f]-7-indolyl,
R² unsubstituiertes oder ein-, zwei- oder dreifach durch A, AO, OH, Hal, CN, NO₂, NH₂, NHA, NA₂, CF₃ COA, CONH₂, COOH, CONHA, CONA₂, OSO₂A und/oder OSO₂CF₃ substituiertes Benzyl oder Phenylhydroxymethyl,
Hal F, Cl, Br, oder I,
A geradkettiges oder verzweigtes Alkyl mit 1-10 C-Atomen, das durch 1 bis 5 F- und/oder Cl-Atome substituiert sein kann oder Cycloalkyl mit 3-10 C-Atomen,
und
m 4
bedeuten, sowie deren Salze.

2. Stereoisomere der Verbindungen der Formel I gemäß Anspruch 1.

3. Verbindungen gemäß der Ansprüche 1 oder 2
a) 3-[4-(3-Benzyl-piperidin-1-yl]-butyl}-indol;
b) 3-[4-(3-Phenyl-hydroxymethyl-piperidin-1-yl)-butyl]-indol;
c) 3-[4-(3-Benzyl-piperidin-1-yl)-butyl]-5-fluor-indol;
d) 3-[4-(3-Benzyl-piperidin-1-yl)-butyl]-5-carboxy-indol;
e) 3-[4-(3-Benzyl-piperidin-1-yl)-butyl]-indol-5-carbonsäuremethylester;
f) (-)-3-{4-[3-(3R')-Benzyl-piperidin-1-yl]-butyl}-indol-5-carbonsäuremethylester;
g) (+)-3-{4-[3-(3S')-Benzyl-piperidin-1-yl]-butyl}-indol-5-carbonsäuremethylester;
h) 3-{4-[3-(3R')-Benzyl-piperidin-1-yl]-butyl}-6-methoxyindol;
i) 3-{4-[3-(3S')-Benzyl-piperidin-1-yl]-butyl}-6-methoxyindol;
j) (+)-3-[4-(3-Benzyl-piperidin-1-yl)-butyl]-indol;
k) (-)-3-[4-(3-Benzyl-piperidin-1-yl)-butyl]-indol;
l) 3-[4-(3-Benzyl-piperidin-1-yl)-butyl]-5-chlorindol;
m) 3-[4-(3-Benzyl-piperidin-1-yl)-butyl]-5-methoxy-indol;
n) 3-{4-[3-(4-Fluorbenzyl)-piperidin-1-yl]-butyl}-5-fluor-indol;
o) 7-{4-[(3R)-3-Benzyl-piperidin-1-yl]-butyl}-5H-1,3-dioxolo[4,5-f]indol;
p) 7-{4-[(3S)-3-Benzyl-piperidin-1-yl]-butyl}-5H-1,3-dioxolo[4,5-f]indol;
q) 3-{4-[(3R)-3-Benzyl-piperidin-1-yl]-butyl}-5-fluorindol;
r) 3-{4-[(3S)-3-Benzyl-piperidin-1-yl]-butyl}-5-fluorindol;
s) 3-{4-[(3R)-3-Benzyl-piperidin-1-yl]-butyl}-indol-5-carbonitril;
t) 3-{4-[(3S)-3-Benzyl-piperidin-1-yl]-butyl}-indol-5-carbonitril;
u) 3-{4-[3-(4-Fluorphenylhydroxymethyl)-piperidin-1-yl]-butyl}-indol-5-carbonitril;
sowie deren Salze.

4. Verfahren zur Herstellung von Verbindungen der Formel I gemäß Anspruch 1, **dadurch gekennzeichnet, dass** man
eine Verbindung der Formel II 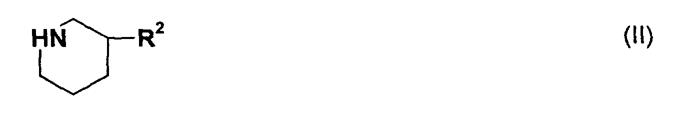 worin R² die in Anspruch 1 angegebene Bedeutung hat, mit einer Verbindung der Formel III
R¹-(CH₂)ₘ - L (III)
worin
L Cl, Br, I, OH, OCOA, OCOPh, OSO₂A, OSO₂Ar, wobei Ar für Phenyl oder Tolyl und A für Alkyl stehen, oder eine andere reaktionsfähig veresterte OH-Gruppe oder leicht nucleophil substituierbare Abgangsgruppe bedeutet
und
R¹ und m die in Anspruch 1 angegebenen Bedeutungen haben,
umsetzt,
oder
in einer reduktiven Aminierung eine Verbindung der Formel IV
R¹ - (CH₂)ₘ₋₁ - CHO (IV)
worin R¹ und m die in Anspruch 1 gegebenen Bedeutungen haben, mit einer Verbindung der Formel (II) umsetzt,
oder dass man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-Cund/oder C-N-Bindung(en) enthält, mit einem reduzierenden Mittel behandelt,
oder dass man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder dass man gegebenenfalls einen Rest R¹ und/oder R² in einen anderen Rest R¹ und/oder R² umwandelt, indem man z.B. eine OA-Gruppe unter Bildung einer OH-Gruppe spaltet und/ oder eine CN-, COOH-, COOA-Gruppe derivatisiert und/oder dass man z.B. ein primäres oder sekundäres N-Atom alkyliert und/oder eine erhaltene Base oder Säure der Formel I durch Behandeln mit einer Säure oder Base in eines ihrer Salze umwandelt.

5. Verfahren zur Herstellung pharmazeutischer Zubereitungen, dadurch gekennnzeichnet, dass man eine Verbindung der Formel I gemäß Anspruch 1 und/oder eines ihrer physiologisch verträglichen Salze, eines ihrer Enantiomere oder Diastereomere der Verbindungen der Formel I zusammen mit mindestens einem festen, flüssigen oder halbflüssigen Träger- oder Hilfsstoff und gegenenenfalls in Kombination mit einem oder mehreren weiteren Wirkstoffen in eine geeignete Dosierungsform bringt.

6. Pharmazeutische Zubereitung, **gekennzeichnet durch** einen Gehalt an mindestens einer Verbindung der allgemeinen Formel I gemäß Anspruch 1 und/oder einem ihrer physiologisch unbedenklichen Salze.

7. Verwendung von Verbindungen der Formel I gemäß Anspruch 1 und/oder von deren physiologisch unbedenklichen Salze zur Herstellung eines Arzneimittels.

8. Verwendung von Verbindungen der Formel gemäß Anspruch 1 und/oder von deren physiologisch unbedenklichen Salze zur Herstellung eines Arzneimittels zur Bekämpfung von Krankheiten.

9. Verwendung von Verbindungen der Formel I gemäß Anspruch 1 und/oder von deren physiologisch unbedenklichen Salze zur Herstellung eines Arzneimittels zur Prophylaxe und/oder Behandlung von Schlaganfällen, Hirn- und Rückenmarkstraumata oder cerebraler Ischämien.

## Claims

1. Compounds of the formula I 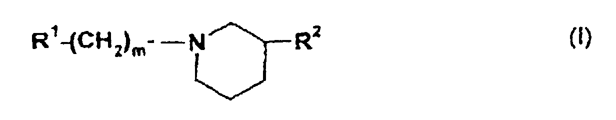 in which
R¹ is 2- or 3-indolyl or 5H-1,3-dioxolo[4,5-f]-7-indolyl, each of which is unsubstituted or mono- or disubstituted by Hal, CN, A, AO, OH, CONH₂, CONHA, CONA₂, COOH and/or COOA,
R² is benzyl or phenylhydroxymethyl, each of which is unsubstituted or mono-, di- or trisubstituted by A, AO, OH, Hal, CN, NO₂, NH₂, NHA, NA₂, CF₃, COA, CONH₂, COOH, CONHA, CONA₂, OSO₂A and/or OSO₂CF₃,
Hal is F, Cl, Br or I,
A is straight-chain or branched alkyl having 1-10 carbon atoms, which may be substituted by from 1 to 5 F and/or Cl atoms, or is cycloalkyl having 3-10 carbon atoms,
and
m is 4,
and salts thereof.

2. Stereoisomers of the compounds of the formula I according to Claim 1.

3. Compounds according to Claims 1 or 2
a) 3-[4-(3-benzylpiperidin-1-yl)butyl]indole;
b) 3-[4-(3-phenylhydroxymethylpiperidin-1-yl)butyl]indole;
c) 3-[4-(3-benzylpiperidin-1-yl)butyl]-5-fluoroindole;
d) 3-[4-(3-benzylpiperidin-1-yl)butyl]-5-carboxyindole;
e) methyl 3-[4-(3-benzylpiperidin-1-yl)butyl]indole-5-carboxylate;
f) methyl (-)-3-{4-[3-(3R')-benzylpiperidin-1-yl]butyl}indole-5-carboxylate;
g) methyl (+)-3-{4-[3-(3S')-benzylpiperidin-1-yl]butyl}indole-5-carboxylate;
h) 3-{4-[3-(3R')-benzylpipieridin-1-yl]butyl}-6-methoxyindole;
i) 3-{4-[3-(3S')-benzylpipieridin-1-yl]butyl}-6-methoxyindole;
j) (+)-3-[4-(3-benzylpiperidin-1-yl)butyl]indole;
k) (-)-3-[4-(3-benzylpiperidin-1-yl)butyl]indole;
l) 3-[4-(3-benzylpiperidin-1-yl)butyl]-5-chloroindole;
m) 3-[4-(3-benzylpiperidin-1-yl)butyl]-5-methoxyindole;
n) 3-{4-[3-(4-fluorobenzyl)pipieridin-1-yl]butyl}-5-fluoroindole;
o) 7-{4-[(3R)-3-benzylpiperidin-1-yl]butyl}-5H-1,3-dioxolo[4,5-f]-indole;
p) 7-{4-[(3S)-3-benzylpiperidin-1-yl]butyl}-5H-1,3-dioxolo[4,5-f]-indole;
q) 3-{4-[(3R)-3-benzylpiperidin-1-yl]butyl}-5-fluoroindole;
r) 3-{4-[(3S)-3-benzylpiperidin-1-yl]butyl}-5-fluoroindole;
s) 3-{4-[(3R)-3-benzylpiperidin-1-yl]butyl}-indole-5-carbonitrile;
t) 3-{4-[(3S)-3-benzylpiperidin-1-yl]butyl}-indole-5-carbonitrile;
u) 3-{4-[3-(4-fluorophenylhydroxymethyl)piperidin-1-yl]butyl}indole-5-carbonitrile;
and salts thereof.

4. Process for the preparation of compounds of the formula I according to Claim 1, **characterised in that**
a compound of the formula II 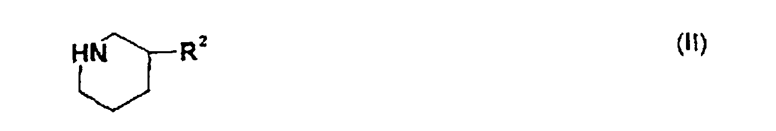 in which R² is as defined in Claim 1, is reacted with a compound of the formula III
R¹-(CH₂)ₘ-L (III)
in which
L is Cl, Br, I, OH, OCOA, OCOPh, OSO₂A, OSO₂Ar, where Ar is phenyl or tolyl and A is alkyl, or another reactively esterified OH group or readily nucleophilically substitutable leaving group,
and
R¹ and m are as defined in Claim 1,
or
in a reductive amination, a compound of the formula IV
R¹-(CH₂)ₘ₋₁-CHO (IV)
in which R¹ and m are as defined in Claim 1, is reacted with a compound of the formula (II),
or **in that** a compound which otherwise conforms to the formula I, but which contains one or more reducible group(s) and/or one or more additional C-C and/or C-N bond(s) instead of one or more hydrogen atoms, is treated with a reducing agent,
or **in that** a compound which otherwise conforms to the formula I, but which contains one or more solvolysable group(s) instead of one or more hydrogen atoms, is treated with a solvolysing agent,
and/or **in that**, if desired, a radical R¹ and/or R² is converted into another radical R¹ and/or R² by, for example, cleaving an OA group with formation of an OH group and/or derivatising a CN, COOH or COOA group and/or **in that**, for example, a primary or secondary N atom is alkylated and/or a resultant base or acid of the formula I is converted into one of its salts by treatment with an acid or base.

5. Process for the preparation of pharmaceutical preparations, **characterised in that** a compound of the formula I according to Claim 1 and/or one of its physiologically tolerated salts, one of its enantiomers or diastereomers of the compounds of the formula I is converted into a suitable dosage form together with at least one solid, liquid or semiliquid excipient or adjuvant and, if desired, in combination with one or more further active ingredients.

6. Pharmaceutical preparation, **characterised by** a content of at least one compound of the general formula I according to Claim 1 and/or one of its physiologically acceptable salts.

7. Use of compounds of the formula I according to Claim 1 and/or of physiologically acceptable salts thereof for the preparation of a medicament.

8. Use of compounds of the formula I according to Claim 1 and/or of physiologically acceptable salts thereof for the preparation of a medicament for combating diseases.

9. Use of compounds of the formula I according to Claim 1 and/or of physiologically acceptable salts thereof for the preparation of a medicament for the prophylaxis and/or treatment of strokes, brain and spinal trauma or cerebral ischaemia.

## Revendications

1. Composés de formule I 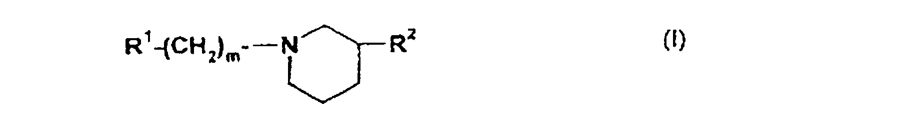 dans laquelle
R¹ représente 2- ou 3-indolyle ou 5H-1,3-dioxolo[4,5-f]-7-indolyle, chacun d'entre eux étant non substitué ou mono- ou disubstitué par Hal, CN, A, AO, OH, CONH₂, CONHA, CONA₂, COOH et/ou COOA,
R² représente benzyle ou phénylhydroxyméthyle, chacun d'entre eux étant non substitué ou mono-, di- ou trisubstitué par A, AO, OH, Hal, CN, NO₂, NH₂, NHA, NA₂, CF₃, COA, CONH₂, COOH, CONHA, CONA₂, OSO₂A et/ou OSO₂CF₃,
Hal représente F, Cl, Br ou I,
A représente alkyle à chaîne linéaire ou ramifiée ayant de 1 à 10 atomes de carbone, pouvant être substitué par de 1 à 5 atomes de F et/ou de Cl, ou représente cycloalkyle ayant de 3 à 10 atomes de carbone,
et
m vaut 4,
et les sels de ceux-ci.

2. Stéréoisomères des composés de formule I selon la revendication 1.

3. Composés selon la revendication 1 ou 2
a) le 3-[4-(3-benzylpipéridin-1-yl)butyl]indole;
b) le 3-[4-(3-phénylhydroxyméthylpipéridin-1-yl)butyl]indole;
c) le 3-[4-(3-benzylpipéridin-1-yl)butyl]-5-fluoroindole;
d) le 3-[4-(3-benzylpipéridin-1-yl)butyl]-5-carboxyindole;
e) le 3-[4-(3-benzylpipéridin-1-yl)butyl]indole-5-carboxylate de méthyle;
f) le (-)-3-{4-[3-(3R')-benzylpipéridin-1-yl]butyl}indole-5-carboxylate de méthyle;
g) le (+)-3-{4-[3-(3S')-benzylpipéridin-1-yl]butyl}indole-5-carboxylate de méthyle;
h) le 3-{4-[3-(3R')-benzylpipéridin-1-yl]butyl}-6-méthoxyindole;
i) le 3-{4-[3-(3S')-benzylpipéridin-1-yl]butyl}-6-méthoxyindole;
j) le (+)-3-[4-(3-benzylpipéridin-1-yl)butyl]indole;
k) le (-)-3-[4-(3-benzylpipéridin-1-yl)butyl]indole;
I) le 3-[4-(3-benzylpipéridin-1-yl)butyl]-5-chloroindole;
m) le 3-[4-(3-benzylpipéridin-1-yl)butyl]-5-méthoxyindole;
n) le 3-{4-[3-(4-fluorobenzyl)pipéridin-1-yl]butyl}-5-fluoroindole;
o) le 7-{4-[(3R)-3-benzylpipéridin-1-yl]butyl}-5H-1,3-dioxolo[4,5-f]-indoie;
p) le 7-{4-[(3S)-3-benzylpipéridin-1-yl]butyl}-5H-1,3-dioxolo[4,5-f]-indole;
q) le 3-{4-[(3R)-3-benzylpipéridin-1-yl]butyl}-5-fluoroindole;
r) le 3-{4-[(3S)-3-benzylpipéridin-1-yl]butyl}-5-fluoroindole;
s) le 3-{4-[(3R)-3-benzylpipéridin-1-yl]butyl}-indole-5-carbonitrile;
t) le 3-{4-[(3S)-3-benzylpipéridin-1-yl]butyl}-indole-5-carbonitrile;
u) le 3-{4-[3-(4-fluorophénylhydroxyméthyl)pipéridin-1-yl]butyl}indole-5-carbonitrile;
et les sels de ceux-ci.

4. Procédé de préparation de composés de formule I selon la revendication 1, **caractérisé en ce que**
un composé de formule II 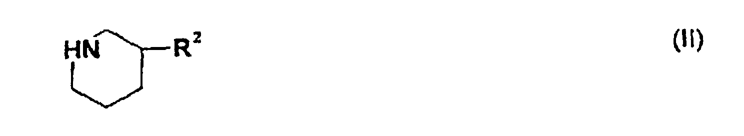 dans laquelle R² est tel que défini selon la revendication 1, est réagi avec un composé de formule III
R¹-(CH₂)ₘ-L (III)
dans laquelle
L représente Cl, Br, I, OH, OCOA, OCOPh, OSO₂A, OSO₂Ar, où Ar représente phényle ou tolyle et A représente alkyle, ou un autre groupement OH estérifié de manière réactive ou groupement partant pouvant être facilement substitué de manière nucléophile,
et
R¹ et m sont tels que définis selon la revendication 1,
ou
dans une amination réductrice, un composé de formule IV
R¹-(CH₂)ₘ₋₁-CHO (IV)
dans laquelle R¹ et m sont tels que définis selon la revendication 1, est réagi avec un composé de formule (II),
ou **en ce qu'**un composé se conformant par ailleurs à la formule I, mais qui contient un ou plusieurs groupement(s) pouvant être réduit(s) et/ou une ou plusieurs liaison(s) C-C et/ou C-N supplémentaire(s) au lieu d'un ou plusieurs atomes d'hydrogène, est traité par un agent réducteur,
ou **en ce qu'**un composé se conformant par ailleurs à la formule I, mais qui contient un ou plusieurs groupement(s) solvolysable(s) au lieu d'un ou plusieurs atomes d'hydrogène, est traité par un agent de solvolysation,
et/ou **en ce que**, si on le désire, un radical R¹ et/ou R² est converti en un autre radical R¹ et/ou R², par exemple, par le clivage d'un groupement OA avec la formation d'un groupement OH et/ou la dérivatisation d'un groupement CN, COOH ou COOA et/ou **en ce que**, par exemple, un atome de N primaire ou secondaire est alkylé et/ou une base ou acide résultant(e) de formule I est converti(e) en l'un de ses sels par traitement par un acide ou une base.

5. Procédé de préparation de préparations pharmaceutiques, **caractérisé en ce qu'**un composé de formule I selon la revendication 1 et/ou l'un de ses sels physiologiquement tolérés, l'un de ses énantiomères ou les diastéréomères des composés de formule I est mis sous une forme de dosage convenable conjointement avec au moins un excipient ou adjuvant solide, liquide ou semi-liquide et, si on le désire, en association avec un ou plusieurs ingrédients actifs supplémentaires.

6. Préparation pharmaceutique, **caractérisée par** une teneur en au moins un composé de formule générale I selon la revendication 1 et/ou l'un de ses sels physiologiquement acceptables.

7. Utilisation de composés de formule I selon la revendication 1 et/ou de sels physiologiquement acceptables de ceux-ci pour la préparation d'un médicament.

8. Utilisation de composés de formule I selon la revendication 1 et/ou de sels physiologiquement acceptables de ceux-ci pour la préparation d'un médicament pour lutter contre des maladies.

9. Utilisation de composés de formule I selon la revendication 1 et/ou de sels physiologiquement acceptables de ceux-ci pour la préparation d'un médicament pour la prophylaxie et/ou le traitement d'accidents vasculaires cérébraux, de traumatismes cérébraux et spinaux ou de l'ischémie cérébrale.
